(19) **Europäisches Patentamt**

**European Patent Office**

**Office européen des brevets**

(11) Numéro de publication: **0 250 475**

**B1**

(12) **FASCICULE DE BREVET EUROPEEN**

(45) Date de publication du fascicule du brevet: **19.09.90**

(51) Int. Cl.⁵: **C 02 F 11/04, C 05 F 7/00, C 05 F 9/00**

(21) Numéro de dépôt: **86906867.6**

(22) Date de dépôt: **04.12.86**

(86) Numéro de dépôt international:
**PCT/FR86/00418**

(87) Numéro de publication internationale:
**WO 87/03575 18.06.87 Gazette 87/13**

(54) **PROCEDE ET INSTALLATION DE RECUPERATION D'ENERGIE A PARTIR DE DECHETS ET RESIDUS.**

(30) Priorité: **05.12.85 FR 8518019**

(43) Date de publication de la demande:
**07.01.88 Bulletin 88/01**

(45) Mention de la délivrance du brevet:
**19.09.90 Bulletin 90/38**

(84) Etats contractants désignés:
**AT BE CH DE GB IT LI LU NL SE**

(56) Documents cités:
**DE-A-3 036 370**
**FR-A-2 240 890**
**FR-A-2 500 990**
**US-A-1 892 681**

**Technische Rundschau, vol. 77, No. 27, July 1985, Hallwag Verlag, (Bern, CH) H. Hauri: "Rauchgasentschwefelung bei mittelgrossen Feuerungsanlagen", pages 28-31, see page 29, "Rauchgasreinigung mit Wärmerückgewinnung"; fig. 2**

(73) Titulaire: **PROPIORGA**
**RN7, La Petite Calade**
**F-13540 Puyricard (FR)**

(72) Inventeur: **NOBILET, Bernard**
**10, le Petit Nice**
**F-13320 Bouc Bel Air (FR)**
Inventeur: **BONHOMME, Michel**
**Les Jardins de Lavalette Bat. A Av. de Vert Bois**
**F-34000 Montpellier (FR)**
Inventeur: **DESPLAT, Philippe**
**La Jipierre Chemin des Plâtrières**
**F-13510 Eguilles (FR)**

(74) Mandataire: **Beauchamps, Georges et al**
**Cabinet Z.Weinstein 20, avenue de Friedland**
**F-75008 Paris (FR)**

**Description**

La présente invention concerne un procédé, et une installation en vue de la mise en oeuvre de ce procédé, permettant la récupération totale de résidus et déchets d'origines diverses notamment les résidus d'origine industrielle ou agricole ou les rejets urbains du type ordures ménagères.

On connaît par le brevet US 1.892.681 une installation pour la production d'énergie à partir de déchets à l'état de boues et dans laquelle les déchets sont soumis à fermentation méthanogène pour la production de gaz, le digestat étant brûlé dans un incinérateur bénéficiant de l'apport de gaz combustible produit dans la phase de méthanogenèse, tandis qu'un apport thermique provenant de l'incinérateur accélère le déroulement de la phase de fermentation méthanogène.

On connaît également par la demande de brevet allemand publiée 3.036.370-, le criblage de résidus bruts et le criblage de la phase solide du digestat.

Le procédé et l'installation selon l'invention permettent de développer les avantages des installations et procédés de ce type en assurant une production d'énergie notamment sous forme de gaz combustible et d'énergie thermique et avec obtention d'un produit à haute valeur ajoutée du type amendement ou engrais organo-minéral; et ceci sans aucun rejet dans la nature de résidus polluants à l'état liquide, solide ou gazeux.

A cet effet l'invention concerne un procédé de récupération et d'exploitation de résidus ou rejets solides d'origine industrielle, agricole ou ménagère, du type dans lequel les résidus sont soumis à une opération de digestion bactérienne pour la production d'un gaz combustible récupéré et stocké, tandis que la phase solide du digestat provenant de l'opération précédente est soumise à incinération dans un four en vue de la production d'énergie thermique, et dans lequel on améliore les conditions de l'incinération en injectant dans le four une partie du gaz provenant de la phase de méthanisation et on améliore les conditions de la production de méthane en utilisant la chaleur du four pour réchauffer le magma en cours de digestion, et le procédé est caractérisé, en outre par la succession des étapes suivantes:

a) on sépare par criblage au sein de la matière de départ les particules les plus grosses qui sont destinées à être acheminées vers le four, des particules les plus fines, qui seront destinées à être acheminées vers le digesteur en vue de la réaction de méthanisation;

b) on mélange la phase desdites particules les plus fines avec une phase liquide constitué des jus séparés du digestat sortant du digesteur de méthanisation.

c) on laisse séjourner les particules les plus fines dans le digesteur de méthanisation, lequel reçoit un apport de calories pour accélérer la réaction, le gaz combustible produit étant stocké dans un gazomètre;

d) on sépare par filtration au sein du digestat sortant du réacteur une phase solide d'une phase liquide constituant les jus, lesquels sont recyclés selon l'étape b) ci-dessus;

e) on soumet la phase solide du digestat à décompactage et criblage, les particules les plus fines formant la phase passante sont séparées tandis que les particules les plus grosses ou refus sont dirigées avec le refus provenant du criblage selon l'étape a) vers le four d'incinération pour constituer le combustible alimentant ledit four;

f) on contrôle à tout moment les paramètres de la combustion au sein dudit four, telle que la température, et on introduit dans le four une quantité dosée de gaz combustible provenant du gazomètre, ceci en fonction des données constatées, pour compléter les insuffisances éventuelles du pouvoir calorifique du combustible solide proncipal en régulant ainsi les conditions de la combustion;

g) on fait passer les fumées de combustion provenant dudit four dans un premier récupérateur de chaleur telle qu'une chaudière tubulaire par exemple ou tous autres échangeurs pour produire un fluide à haute température à des fins industrielles;

h) on lave les fumées au sortir du premier récupérateur de chaleur par neutralisation au moyen d'un lait basique provenant des cendres de combustions mises en suspension dans une phase aqueuse;

i) on recueille ledit lait au sortir de l'étape de neutralisation selon h) ci-dessus et on sépare la phase solide, laquelle est mélangée à la phase passante provenant du criblage selon l'étape e) ci-dessus, pour constituer un amendement organo-minéral pour les terres;

j) les gaz épurés sortant de l'étape de neutralisation selon h) ci-dessus sont acheminés dans un second récupérateur de chaleur débitant un fluide caloporteur véhiculé vers un échangeur de chaleur situé au sein du réacteur de méthanisation en constituant ainsi l'apport de calories de l'étape c) ci-dessus.

L'invention concerne également une installation permettant la mise en oeuvre des diverses caractéristiques du procédé évoqué ci-dessus, et caractérisée en ce qu'elle comporte:

un digesteur 5 de fermentation anaérobie alimentée en déchets ou résidus solides d'origine industrielle, agrocile ou ménagère, en vue de la production de méthane;

un gazomètre 7 formant enceinte de stockage pour le méthane ainsi produit;

des moyens de décantation 8 du digestat provenant du digesteur pour séparer la phase solide d'une phase liquide.

des moyens de criblage 10 disposés en aval du digesteur et desdits moyens de décantation, afin d'assurer la séparation au sein de la phase solide du digestat, des particules les plus fines, et des éléments les plus grossiers ou refus;

un convoyeur apte à véhiculer lesdits refus constituant le combustible destiné à incinération;

un four à incinération 53 pourvu d'une chambre de post-combustion 17 et comportant au moins un brûleur à gaz alimenté depuis ledit gazomètre;

un premier récupérateur de chaleur 19 alimenté en gaz de combustion provenant du four d'incinération et débitant un fluide caloporteur à haute température, et l'installation est caractérisée en ce qu'elle comporte en outre,

un second récupérateur de chaleur tel qu'un condenseur 25 situé en aval du premier récupérateur 19, sur le circuit des fumées et associé à un circuit de fluide caloporteur à moyenne température;

un échangeur de chaleur 38' incorporé au digesteur 5 et relié au second récupérateur de chaleur et ainsi apte à tranférer des calories provenant dudit second récupérateur de chaleur vers le magma en cours de fermentation bactérienne au sein du digesteur;

un bac 21 de préparation d'un lait de neutralisation constitué des cendres de combustion provenant du four d'incinération et mises en suspension dans une phase liquide;

une chambre de neutralisation traversée d'une part par ledit lait de neutralisation et d'autre part par les fumées sortant du premier récupérateur de chaleur 19 et acheminées vers le second récupérateur de chaleur 25, la rencontre des fumées acides et du lait contenant les cendres en suspension étant apte à assurer la neutralisation desdites fumées;

un mélangeur 12 apte à recevoir les particules passantes au niveau du criblage 10 situé en aval du digesteur d'une part, et les extraits solides sédimentés dans le bac de neutralisation 21, ce mélangeur étant apte à dépiter une composition organo-minérale à usage d'amendement ou engrais agricoles ou horticoles 33.

D'autres caractéristiques et avantages de l'invention ressortiront de la description qui suit et qui est donnée en rapport avec une forme particulière présentée à titre d'exemples non limitatifs et à la lumière des dessins annexés.

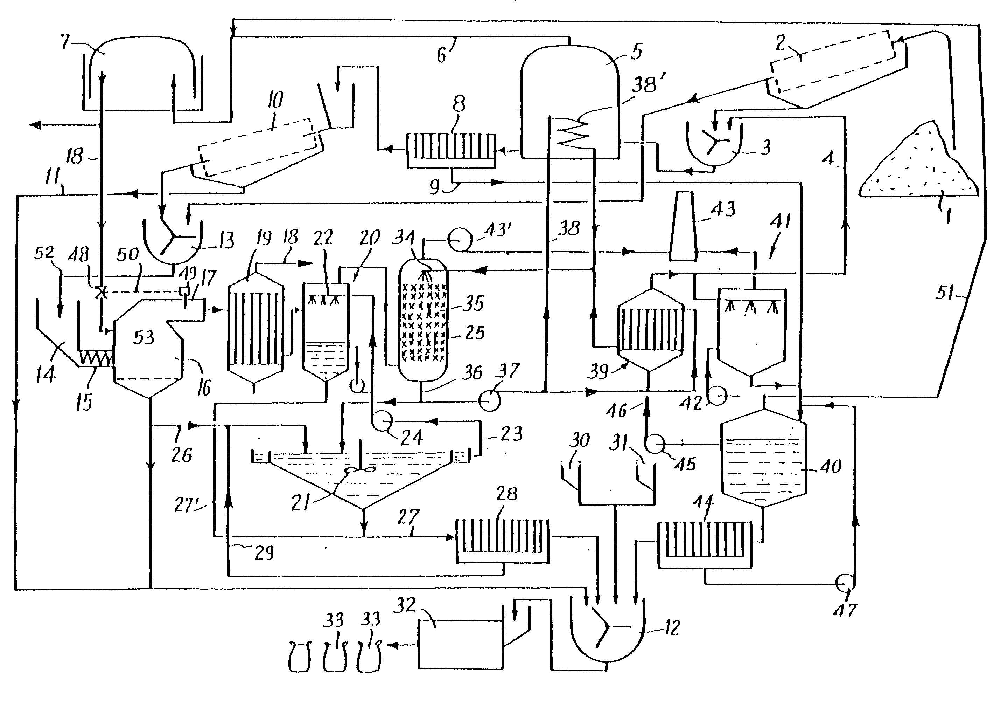

La figure représente une vue d'un schéma de principe du fonctionnement du procédé et de l'installation selon l'invention.

La matière première est prise au tas 1 et acheminée vers un ensemble de criblage ou de séparation constitué éventuellement comme indiqué à titre d'exemple dans la figure, d'un premier crible à tambour rotatif 2; la partie destinée à constituer le produit soumis à digestion est recueilli dans le mélangeur 3 où elle reçoit par la canalisation 4 les jus recyclés et prélevés en aval du digesteur et constituant la phase liquide du magna formant l'éfluent du digesteur ou digestat. Le circuit de recyclage des jus extraits du digestat étant décrit ultérieurement.

La matière première sortant du mélangeur 3 est acheminée par des moyens de convoyage jusqu'au réacteur de méthanisation ou digesteur 5.

Le gaz recueilli au sommet du digesteur est acheminé par la canalisation 6 jusqu'au gazomètre 7 de stockage.

Le magma sortant du réacteur de méthanisation passe dans un appareil de séparation par exemple un filtre presse 8 d'où la phase liquide ou jus est évacuée pur être acheminée par la canalisation 9 vers l'ensemble de concentration décantation des jus décrit ultérieurement.

La phase solide du digestat formée de blocs compacts au sortir de l'appareil de séparation par exemple du filtre presse 8 est émottée de façon à retrouver un état particulaire et à permettre le passage dans un second ensemble de criblage constitué par exemple d'un crible rotatif 10.

Les particules les plus fines ou particules passantes sont recueillies pour constituer la base d'un engrais organominéral et elles sont acheminées par un circuit de convoyage 11 jusqu'au mélangeur 12. Les particules les plus grossières ou refus sortant du crible 10 sont destinées à constituer la matière combustible alimentant l'étape ultérieure ou étape d'incinération.

Le refus provenant de l'appareil de séparation 10 par exemple un crible est à cet effet homogénéisé dans le mélangeur 13 avec le refus provenant du premier ensemble de criblage ou séparation 2 et constitué également de particules plus grossières.

Le combustible provenant du refus des ensembles de séparation est ainsi constitué de particules soit refusées à la première séparation et n'ayant pas transité par le digesteur soit de particules provenant du digestat mais restées à un état granulométrique grossier.

Cet ensemble se compose de débris particulaires notamment morceaux de papier ou carton non totalement digérés dans le digesteur, débris de bois du d'éléments végétaux, débris de matière plastique, os, etc...

Ce matériau possède un pouvoir calorifique intéressant et surtout grâcee au fort pourcentage de produits extraits du digesteur, ce produit possède une homogénéite et des caractéristiques contrôlables de sorte qu'il constitue un combustible utile permettant la régularité et le contrôle de l'alimentation de l'ensemble d'incinération.

Ceci permettra un fonctionnement régulier du four d'incinération s'opposant au fonctionnement par vague et par soubresaut des fours à incinération alimentés en déchets "tout-venant" dont les caractéristiques sont fortement irrégulières et dépourvues de toute homogénéité en ce que concerne la composition, la granulométrie, l'hygrométrie, et partant le pouvoir calorifique.

On pourra à cet effet prévoir que pratiquement l'ensemble des résidues traités depuis le tas de départ 1 passera par la phase de méthanisation afin d'obtenir la plus grande régularité et un contrôle constant dans les caractéristiques du combustible ainsi obtenues.

Les déchets formant le combustible traité dans le mélangeur 13 est acheminé vers la trémie d'alimentation 14 desservant un convoyeur d'alimentation par exemple du type à vis d'archimède 15 ou tout autre appareil contrôlant ainsi l'alimentation du four à incinération 16.

La combustion dans ce four est contrôlée de manière à respecter des consignes de température à chaque étage de leur oxydation par le présence de brûleurs et de dispositifs de régulation de l'air comburant.

Ce four comporte une chambre de post-combustion 17 et des brûleurs 53 à gaz et alimentés depuis le circuit 18 en méthane provenant du gazomètre de stockage 7.

Le four à incinération peut être pourvu de sondes 49 propres à analyser en permanence les paramètres de fonctionnement tels que températures dans les différentes zones du four, composition des fumées etc... et en fonction de ces paramètres l'alimentation en gaz d'appoint depuis la canalisation 18 peut être ajustée de façon à pallier et à compenser les légères sautes de fonctionnement qui peuvent subsister compte tenu des caractéristiques du combustible d'alimentation.

L'appart d'un combustible d'appoint disponible sur place permet d'assurer l'automatisation et le fonctionnement permanent de l'ensemble de l'installation en dehors de la présence de tout personnel de service hors d'une équipe très limité de surveillance et de maintenance.

L'alimentation en gaz combustible d'appoint peut être régulée par une vanne automatique 48 asservie à une sonde 49 par un circuit 50.

Les gas de combustion sortant de la chambre de post-combustion 17 à une température de l'ordre de 900 à 1000°C passent dans un premier récupérateur de chaleur formé par exemple d'une chaudière tubulaire 19 produisant en 18 un fluide caloporteur à haute température tel qu'une vapeur sèche ou de l'eau surchauffée ou tout autre fluide thermique; ce liquide caloporteur est dirigé vers les utilisations possibles telles qu'une turbine de production d'énergie électrique ou toute utilisation conventionnelle.

Les gaz de combustion sortant du récupérateur 19 à une température de l'ordre de 250 à 350°C ne peuvent être rejetés dans l'atmosphère car ils sont fortement chargés en anhydrides ou vapeurs acides (NO2, HCl et SO3) ces produits nocifs provenant généralement de la décomposition et de la combustion des matières plastiques et généralement des matières synthétiques (notamment PVC).

Il est donc impossible de réjeter ces fumées dans l'atmosphère; par ailleurs il est souhaitable de récupérer la chaleur latente et la chaleur sensible contenues dan ces fumées qui au sortir de la chaudière 19 sont encore à une température élevée.

L'installation ici décrite comporte un ensemble de lavage des fumées en vue de leur neutralisation à haute température avant que ces fumées soient refroidies pour atteindre le point de rosée, au-delà duquel seraient formés les acides corrosifs et agressifs pour les métaux constituant les enceintes de l'installation.

les cendres de combustion sont ainsi déversées dans le bac 21 où elles sont mises en suspension ou en solution pour constituer un lait à pH fortement basique lequel peut être prélevé depuis par exemple une goulotte périphérique ou tout autre système par le circuit 23 aboutissant à la rampe de pulvérisation 22 de la chambre de neutralisation 20.

Au sein de la chambre 20 la phase liquide formée du lait basique ci-dessus décrit rencontre les vapeurs acides qui sont neutralisées en aboutissant à la formation de sels généralement insolubles lesquels sont sédimentés pour aboutir par les circuits d'évacuation 27, 27' au filtre presse 28; ces sels après essorage sont déversés dans le mélangeur 12 où ils rencontrent les particules fines provenant du digestat et constituent la base de l'engrais organo-minéral.

L'incorporation à ces particules fines constituant un amendement, des principes minéraux provenant de l'ensemble de neutralisation enrichit la base de l'amendement en principes actifs notamment azote, phosphore et potassium et permet d'obtenir ainsi un amendement riche ou un véritable engrais organo-minéral.

Ce dernier peut en fonction des ajustements nécessaires recevoir l'incorporation d'additifs provenant des trémies 30 et 31 pour obtenir une richesse constante en principes actifs.

L'ensemble mélangé et homogène est acheminé depuis le mélangeur 12 vers la trémie d'alimentation d'un ensemble d'ensachage 32 pour être débité en doses conditionnées sous forme de sacs d'engrais organo-minéral 33, 33'.

Les gaz épurés sortant de la chambre de neutralisation 20 et fortement chargés en vapeur d'eau peuvent alors être soumis à une opération de condensation dans la tour de condensation ou condenseur 25 où ils rencontrent à contrecourant une eau de ruissellement provenant du ou des pulvérisateur (s) supérieur (s) 34.

L'eau de ruissellement, enrichie de l'eau de condensation provenant des gaz épurés est recueillie chaude à la base de la colonne 25 d'où elle est partiellement recyclée vers le bac 21 tandis que la majeure partie est acheminée depuis le circulateur 37 vers les échangeurs de chaleur secondaires respectivement 38' et 39 par le circuit secondaire 38.

L'éfluent provenant de la tour de condensation 25 est en effet une eau pure portée à une température de l'ordre de 60°; sa chaleur sensible est récupérée dans les échangeurs de chaleur respectivement le premier échangeur de chaleur sécondaire 38' situé au sein du magma en cours de traitement dans le digesteur 5 et cédant ses calories à ce magma en cours de fermentation, et le second échangeur de chaleur secondaire 39 appartenant à l'ensemble de condensation de décantation des jus provenant par la canalisation 9 de la filtration en 8 du digestat.

L'échangeur de chaleur 39 cède ses calories au jus de filtration du digestat stocké dans le réservoir tampon 40 lui-même alimenté par la canalisation 9; le jus réchauffé pulvérisé au sommet de l'évaporateur 41 où il rencontre le courant ascendant d'air frais provenant du ventilateur 42, l'air chargé d'humidité étant évacué au sommet de l'évaporateur 41 pour être dirigé éventuellement vers la cheminée de tirage 43.

Le réservoir tampon 40 peut être agencé pour constituer un fermentateur secondaire alimenté en jus concentrés et réchauffés et permettant une production de méthane rejoignant par le circuit 51 le réservoir de stockage 7.

Les jus ainsi concentrés et ayant éventuellement subis une fermentation complémentaire sont recyclés par le circuit 4 vers le mélangeur 3 pour l'alimentation du digesteur 5 tandis qu'une partie des jus est renvoyée au réservoir tampon 40.

Ce réservoir tampon comporte éventuellement des moyens permettant le prélèvement à sa base des particules solides sédimentées et qui après concentration dans l'appareil de séparation par exemple le filtre presse 44 sont envoyées dans le mélangeur 12 pour enrichir l'engrais organo-minéral, notamment en matière azotée, la phase liquide de filtration étant recyclée par la pompe 47 vers le réservoir 40.

Selon un développement de l'invention les éfluents gazeux sortant du digesteur 5 et/ou du fermenteur secondaire 40 sont épurés avant de rejoindre le gazomètre 7 afin de séparer une première phase riche en méthane d'une seconde phase constituée principalement d'air et d'oxyde de carbone; cette dernière phase n'est cependant pas rejetée dans l'atmosphère car elle contient encore un faible pourcentage (de l'ordre de 4 à 5%) de méthane; cette seconde phase est donc injectée dans le brûleur à incinération 16 où l'air qu'elle contient constitue le gaz comburant tandis que le méthane et éventuellement le monoxyde de carbone sont récupérés et brûlés, complètant ainsi l'épuisement de l'énergie thermique potentielle contenue dans la matière première de départ.

**Revendications**

1. Procédé de récupération et d'exploitation de résidus ou rejets solides d'origine industrielle, agricole ou ménagère, du type dans lequel les résidues sont soumis à une opération de digestion bactérienne pour la production d'un gaz combustible récupéré et stocké, tandis que la phase solide du digestat provanant de l'opération précédente est soumise à incinération dans un four en vue de la production d'énergie thermique, et dans lequel on améloire les conditions de l'incinération en injectant dans le four une partie du gaz provenant de la phase de méthanisation et on améloire les conditions de la production de méthane en utilisant la chaleur du four pour réchauffer le magma en cours de digestion, et le procédé est caractérisé, en outre par la succession des étapes suivantes:

a) on sépare par criblage au sein de la matière de départ les particules les plus grosses qui sont destinées à être acheminées vers le four, des particules les plus fines, qui sont destinées à être acheminées vers le digesteur en vue de la réaction de méthanisation;

b) on mélange la phase desdites particules les plus fines avec une phase liquide constituée des jus séparés du digestat sortant du digesteur de méthanisation.

c) on laisse séjourner les particules les plus fines dans le digesteur de méthanisation, lequel reçoit un apport de calories pour accélérer la réaction, le gaz combustible produit étant stocké dans un gazomètre;

d) on sépare par filtration au sein du digestat sortant du réacteur une phase solide d'une phase liquide constituant les jus, lesquels sont recyclés selon l'étape b) ci-dessus;

e) on soumet la phase solide du digestat à décompactage et criblage, les particules les plus fines formant la phase passante sont séparées tandis que les particules les plus grosses ou refus sont dirigées avec le refus provenant du criblage selon l'étape a) vers le four d'incinération pour constituer le combustible alimentant ledit four;

f) on contrôle à tout moment les paramètres de la combustion au sein dudit four, telle que la température, et on introduit dans le four une quantité dosée de gaz combustible provenant du gazomètre, ceci en fonction des données constatées, pour compléter les insuffisances éventuelles du pouvoir calorifique du combustible solide principal en régulant ainsi les conditions de la combustion;

g) on fait passer les fumées de combustion provenant dudit four dans un premier récupérateur de chaleur telle qu'une chaudière tubulaire par exemple ou tous autres échangeurs pour produire un fluide à haute température à des fins industrielles;

h) on lave les fumées au sortir du premier récupérateur de chaleur par neutralisation au moyen d'un lait basique provenant des cendres de combustions mises en suspension dans une phase aqueuse;

i) on recueille ledit lait au sortir de l'étape de neutralisation selon h) ci-dessus et on sépare la phase solide, laquelle est mélangée à la phase passante provenant du criblage selon l'étape e) ci-dessus, pour constituer un amendement organo-minéral pour les terres;

j) les gaz épurés sortant de l'étape de neutralisation selon h) ci-dessus sont acheminés dans un second récupérateur de chaleur débitant un fluide caloporteur véhiculé vers un échangeur de chaleur situé au sein du réacteur de méthanisation en constituant ainsi l'apport de calories de l'étape c) ci-dessus.

2. Procédé selon la revendication 1 ci-dessus, caractérisé en ce que l'énergie thermique provenant de la phase d'incinération et véhiculée par le fluide caloporteur provenant du second récupérateur de chaleur est utilisé pour obtenir la concentration des jus provenant de la séparation de la phase solide du digestat, en vue du recyclage desdits jus après concentration, lesquels sont incorporés dans la matière de départ introduite dans le digesteur.

3. Procédé selon la revendication 1 ci-dessus, caractérisé en ce outre en ce que l'énergie thermique provenant de la phase d'incinération et contenue dans le fluide caloporteur provenant du second récupérateur de chaleur est utilisée pour réchauffer et concentrer les jus liquides séparés du digestat, lesquels sont introduits en phase liquide dans un second digesteur ou digesteur secondaire pour la production d'un flux auxiliaire de méthane.

4. Procéde selon la revendication 1 ci-dessus, caractérisé en ce que les particules les plus fines formant la phase passante de ladite étape de criblage e) ci-dessus, sont dirigées vers un mélangeur dans lequel elles sont incorporées aux résidus solides provenant d'une phase de sédimentation du lait de neutralisation formé des cendres mises en suspension en phase aqueuse, ces produits au sein du mélangeur recevant en outre les résidus solides provenant de la décantation des boues extraites du digestat pour constituer par l'incorporation de ces trois éléments un amendement ou engrais horticole organo-minéral.

5. Procédé selon la revendication 4 ci-dessus, caractérisé en ce que les résidus et rejets traités sont en outre soumis à une étape d'élimination des solides constitués des objets ou éléments en verre, cette phase verrière étant soumise à concassage jusqu'à obtention d'une granulométrie pulvérulente aboutissant à la formation d'une phase sableuse laquelle est introduite dans ledit mélangeur (12) pour obtenir un substrat de culture complet.

6. Installation pour la mise en oeuvre du procédé selon l'une des revendications 1 à 5, ci-dessus, et du type comportant:

un digesteur (5) de fermentation anaérobie alimentée en déchets ou résidus solides d'origine industrielle, agricole ou ménagère, en vue de la production de méthane;

un gazomètre (7) formant enceinte de stockage pour le méthane ainsi produit;

des moyens de décantation (8) du digestat provenant du digesteur pour séparer la phase solide d'une phase liquide.

des moyens de criblage (10) disposés en aval du digesteur et desdits moyens de décantation, afin d'assurer la séparation au sein de la phase solide du digestat, des particules les plus fines, et des éléments les plus grossiers ou refus;

un convoyeur apte à véhiculer lesdits refus constituant le combustible destiné à incinération;

un four à incinération (16) pourvu d'une chambre de post-combustion (17) et comportant au moins un brûleur (53) à gaz alimenté depuis ledit gazomètre;

un premier récupérateur de chaleur (19) alimenté en gaz de combustion provenant du four d'incinération et débitant un fluide caloporteur à haute température, et l'installation est caractérisée en ce qu'elle comporte en outre,

un second récupérateur de chaleur tel qu'un condenseur (25) située en aval du premier récupérateur (19), sur le circuit des fumées et associé à un circuit de fluide caloporteur à moyenne température;

un échangeur de chaleur (38') incorporé au digesteur (5) et relié au second récupérateur de chaleur et ainsi apte à transférer des calories provenant dudit second récupérateur de chaleur vers le magma en cours de fermentation bactérienne au sein du digesteur;

un bac (21) de préparation d'un lait de neutralisation constitué des cendres de combustion provenant du four d'incinération et mises en suspension dans une phase liquide;

une chambre de neutralisation traversée d'une part par ledit lait de neutralisation et d'autre part par les fumées sortant du premier récupérateur de chaleur (19) et acheminées vers le second récupérateur de chaleur (25), la rencontre des fumées acides et du lait contenant les cendres en suspension étant apte à assurer la neutralisation desdites fumées;

un mélangeur (12) apte à recevoir les particules passantes au niveau du criblage (10) situé en aval du digesteur d'une part, et les extraits solides sédimentés dans le bac de neutralisation (21), ce mélangeur étant apte à débiter une composition organo-minérale à usage d'amendement ou engrais agricoles ou horticoles (33).

7. Installation selon la revendication 6 ci-dessus, caractérisé en ce qu'elle comporte un ensemble de concentration ou décantation des jus provenant de la phase de filtration (9) au sortir du digesteur et cet ensemble comorte un échangeur de chaleur (39) alimenté par le fluide coloporteur provenant du second récupérateur de chaleur (25), cet échangeur (39) étant relié par une canalisation d'arrivée (9) auxdits moyens de filtration (8) situé en aval du digesteur (5), et par une canalisation de sortie (4) aboutissant à l'alimentation (3) du digesteur en vue de recycler les jus concentrés pour alimenter et ensemencer le digesteur.

8. Installation selon la revendication 7 ci-dessus, caractérisé en ce que ledit ensemble de concentration des jus comporte en outre un réservoir tampon (40) apte à assurer la décantation desdits jus, ce réservoir étant alimenté par la canalisation (9) depuis la filtration (8) au sortir du digesteur et le réservoir tampon (40) alimente ledit échangeur de chaleur (39) pour provoquer le réchauffement des jus, l'installation comportant en outre une enceinte d'évaporation (41) dans laquelle les jus réchauffés s'écoulent à contrecourant d'un flux d'air frais qui se charge en vapeur, les jus concentrés étant recueillis à la base de l'évaporateur (41) et acheminés par une canalisation de recyclage audit réservoir tampon (40), la base du réservoir tamponn (40) comportant des moyens d'extraction des dépôts solides sédimentés et reliés par des moyens de convoyage audit mélangeur (12) en vue de l'incorporation desdits dépôts sédimentés dans l'engrais organo-minéral, et ledit réservoir tampon (40) apte à assurer un réaction de digestion en phase liquide desdits jus, est relié par son sommet à un circuit d'évacuation du méthane produit en son sein vers le gazomètre de stockage (7).

9. Installation selon l'une des revendications 6 à 8, caractérisée en ce que ledit mélangeur (12) apte à assurer l'homogénéité de l'engrais organo- minéral (33) comporte en outre au moins une trémie (30, 31) contenant une réserve d'additif tel qu'un sel minéral propre à ajuster la composition du mélange en vue d'assurer la richesse constante de l'engrais en principes actifs.

**Patentansprüche**

1. Verfahren zur Rückgewinnung und Verwertung von festen Industrie-Landwirtschafts- oder Haushaltsabfällen, bei dem die Abfälle einem bakteriellen Gärungsprozess zur Erzeugung eines zurückgewonnenen und gespeicherten Gases unterworfen werden, während die Feststoffphase des Gärungsproduktes, das von dem vorangehenden Prozess herrührt, in einem Ofen verbrannt wird, um Wärmeenergie zu erzeugen, und bei dem die Verbrennungsbedingungen durch Einblasen eines Teiles des Gases, das von der Methanisierungsphase herrührt, verbessert werden, und die Bedingungen für die Erzeugung von Methan durch Verwendung der Wärme des Ofens verbessert werden, um den in Gärung befindlichen Brei zu erwärmen, wobei das Verfahren ferner durch die Folge der folgenden Arbeitsschritte gekennzeichnet ist:

a) man trennt durch Sieben im Inneren des Ausgangsmaterials die grössten Teilchen, die dazu bestimmt sind, zum Ofen gefördert zu werden, von den kleinsten Teilchen, die zur Förderung zum Gärapparat zum Zwecke der Methanisierungsreaktion bestimmt sind;

b) man mischt die Phase der genannten kleinsten Teilchen mit einer Flüssigphase, die von den Säften gebildet wird, die auch von dem Gärgut abgetrennt worden sind, dass aus dem Methanisationsgärapparat austritt;

c) man lässt die kleinsten Teilchen in dem Methanisationsgärapparat verbleiben, der eine Zufuhr von Kalorien zur Beschleunigung der Reaktion erhält, wobei das erzeugte Brenngas in einem Gasometer gespeichert wird;

d) man trennt durch Filtern im Inneren des Gärungsgutes, das aus dem Reaktor austritt, eine Feststoffphase von einer Flüssigphase, die die Säfte bildet, die gemäss dem obigen Arbeitsschritt b) wieder in den Kreislauf zurückgeführt werden;

e) man unterwirft die Feststoffphase des Gärungsgutes einer Dekompaktierung oder einem Sieben, wobei die feinsten Teilchen, die die Fliessphase bilden, abgetrennt werden, während die grössten Teilchen oder die Abfälle, die mit dem Abfall, der vom Sieben gemäss dem Schritt a) herrühren, zum Verbrennungsofen gebracht werden, um den Brennstoff zu bilden, der den Ofen speist;

f) man kontrolliert zu jedem Zeitpunkt die Parameter der Verbrennung im Inneren den Ofens, wie die Temperatur, und gibt in den Ofen eine dosierte Menge von Brenngas ein, das vom Gasometer herkommt, und dies in Abhängigkeit der festgestellten Daten, um die eventuellen Mängel der Wärmeenergie des festen Hauptbrennstoffs zu vervollständigen, indem man so die Verbrennungsbedingungen reguliert;

g) man überführt die vom Ofen kommenden Verbrennungsgase in einem ersten Wärmeaustauscher wie zum Beispiel einen rohrförmigen Kessel oder beliebig andere Wärmeaustauscher um ein Hochtemperaturfluid für industrielle Verwendungszwecke zu erzeugen;

h) man wäscht die aus dem ersten Wärmeaustauscher austretenden Gase durch Neutralisation mit Hilfe einer basischen Milch, die von den Verbrennungsaschen herrührt, die in einer wässerigen Phase in Suspension gebracht sind;

i) man sammelt die genannte Milch am Ausgang der Neutralisationsphase gemäss obigem h) und trennt die Feststoffphase ab, die mit der Fliessphase vermischt wird, die von dem Sieben gemäss obigem Arbeitsschritt e) herrührt, um einen organisch-mineralischen Zusatzstoff für die Erden zu bilden;

j) die gereinigten, aus dem Neutralisationsarbeitsschritt gemäss obigem h) austretenden Gase werden in einen zweiten Wärmeaustauscher geführt, der ein Wärmeütransportfluid abgibt, das zu einem Wärmeaustauscher geführt wird, der sich im Inneren des Methanisationsreaktors befindet, indem man auf diese Weise die Wärmekalorienzufuhr des obigen Arbeitsschritts c) bewirkt.

2. Verfahren nach dem obigen Anspruch 1, dadurch gekennzeichnet, dass die Wärmeenergie, die von der Verbrennungsphase herrührt und durch das Wärmeütransportfluid, das von dem zweiten Wärmeaustauscher herrührt, gefördert wird, verwendet wird, um die Konzentration der Säfte, die von der Trennung der Feststoffphase des Gärgutes herrühren, zum Zwecke der Rückführung der genannten Säfte nach der Konzentration, die in das Ausgangsgut, das in den Gärapparat eingeführt worden ist, eingebracht worden sind.

3. Verfahren nach obigem Anspruch 1, dadurch gekennzeichnet, dass ferner die Wärmeenergie, die von der Verbrennungsphase herrührt und in dem Wärmeütransportfluid enthalten ist, das von dem zweiten Wärmeaustauscher herkommt, zum Erwärmen und zur Konzentration der flüssigen, vom Gärgut abgetrennten Säfte verwendet wird, die als Flüssigphase in einen zweiten Gärapparat oder sekundären Gärapparat zur Erzeugung eines Methanhilfsflusses eingebracht werden.

4. Verfahren nach obigem Anspruch 1, dadurch gekennzeichnet, dass die feinsten Teilchen, die die Fliessphase des obigen Siebsschrittes e) bilden, zu einem Mischer geführt werden, in dem sie den festen Abfällen, die von einer Sedimentationsphase der Neutralisationsmilch herrühren, die aus der in der Flüssigphase suspendierten Asche gebildet wird, einverleibt werden, wobei diese Produkte im Inneren des Mischers, der ferner die festen Abfälle aufnimmt, die von dem Dekantieren des den Gärgut entzogenen Schlammes herrühren, um durch Einverlieben dieser drei Elemente einen organisch-mieralischen Zusatzstoff oder Düngemittel für den Gartenbau zu bilden.

5. Verfahren nach obigem Anspruch 4, dadurch gekennzeichnet, dass die behandelten Abfälle und Auschüsse ferner einem Arbeitsschritt zum Entfernen der Feststoffe, die von Gegenständen oder Elementen aus Glas gebildet werden, unterworfen werden, wobei diese Glasphase einem Zerkleinerungsschritt bis zum Erhalt einer pulverförmigen Korngrösse unterworfen werden, die zu Bildung einer sandförmigen Phase führt, die in den genannt Mischer (12) zum Erhalt eines vollständigen Pflege- oder Bebauungssubstrats eingegeben wird.

6. Einrichtung zur Ausführung des Verfahrens nach einem der obigen Ansprüche 1 bis 5, die besitzt:

einen Gärapparat (5) zur anaerobischen Vergärung, der mit festen Industrie- Landwirtschafts- oder Haushaltsabfällen zur Erzeugung von Methan gespeist wird;

einen Gasometer (7), der einen geschlossenen Speicherraum für das so erzeugte Methan bildet;

Mittel (8) zum Dekantieren des Gärgutes, das vom Gärapparat herrüht, um die Feststoffphase von einer Flüssigphase zu trennen;

Siebmittel (10), die vor dem Gärapparat und den genannten Dekantierungsmitteln angeordnet sind, um die Trennung im Inneren der Festkörperphase der kleinsten Teilchen und der grössten Teilchen oder dem Ausschuss zu gewährleisten;

einen Förderer, der geeignet ist, um die genannten Ausschüsse, die den Brennstoff für die Verbrennung bilden, zu fördern;

einen Verbrennungsofen (16) mit einer Nachbrennkammer (17) und wenigstens einem Gasbrenner (53), der von dem genannten Gasometer gespeist wird;

einen ersten Wärmetauscher (19), der mit Verbrennungsgas gespeist wird, das vom Verbrennungsofen herkommt und ein Wärmeütransportfluid hoher Temperatur abgibt, wobie die Anlage dadurch gekennzeichnet ist, dass sie ferner umfasst:

einen zweiten Wärmeaustauscher, wie einen Kondensator (25), der hinter dem ersten Wärmeaustauscher (19) im dem Kreislauf der Abgase angeordnet ist und einem Wärmemitteltransportfluidkreis mittlerer Temperatur zugeordnet ist;

einen Wärmeaustauscher (38'), der in dem Gärapparat (5) angeordnet ist und mit dem zweiten Wärmeaustauscher verbunden ist und infolgedessen geeignet ist, die Kalorien, die vom zweiten Wärmeaustauscher herrühren, zu dem im bakteriellen Vergären befindlichen Brei im Inneren des Gärapparates zu fördern;

einen Behälter (21) zur Zubereitung einer Neutralisationsmilch, die von der Verbrennungsasche gebildet wird, die vom Verbrennungsofen herrührt und in einer Flüssigphase in Suspension ist;

eine Neutralisationskammer, die einerseits von der genannten Neutralisationsmilch und andererseits von den Abgasen durchströmt wird, die aus den ersten Wärmeaustauscher (19) austreten und zu dem zweiten Wärmeaustauscher (25) geführt werden, wobei das Zusammentreffen der sauren Abgase und der die Suspensionsasche enthaltenden Milch geeignet ist, die Neutralisation der genannten Abgase zu bewirken;

einen Mischer (12), der geeignet ist, die fliessenden Teilchen im Bereich des Siebens (10, hinter dem Gärapparat einerseits, und die festen Auszüge, die sich in dem Neutralisationsbehälter (21) abgesetzt haben, zu empfangen, wobei der Mischer geeignet ist, eine organischemineralische Komposition zum Gebrauch als Zusatz oder Düngemittel für die Landwirtschaft oder den Gartenbau (33) liefern.

7. Einrichtung nach obigem Anspruch 6, dadurch gekennzeichnet, dass sie einen Komplex zur Konzentration oder zum Dekantieren der Säfte, die von der Filtrierungsphase (9) am Ausgang des Gärapparates herrühren, besitzt und dieser Komplex einen Wärmeaustauscher (39) besitzt, der mit dem Wärmeütransportfluid, das von dem zweiten Wärmeaustauscher (25) herrührt, gespeist wird, wobei dieser Wärmeaustauscher (39) durch eine Zufuhrleitung (9) mit den genannten Filtrierungsmitteln (8), die stromabwärts vom Gärapparat (5) angeordnet sind, und durch eine Ausgangsleitung (4) mit der Speisung (3) des Gärapparates zur Zurückführung der konzentrierten Säfte zur Speisung und zum Impfen des Gärapparates verbunden ist.

8. Einrichtung nach dem obigen Anspruch 7, dadurch gekennzeichnet, dass der genannte Komplex zur Konzentration der Säfte ferner einen Pufferbehälter (40) besitzt, der geeignet ist, die Dekantierung der genannten Säfte zu gewährleisten, wobei der Behälter durch die Leitung (9) von der Filtration am Ausgang des Gärapparates gespeist wird, und der Pufferbehälter (40) den genannten Wärmeaustauscher (39) speist, um eine Erwärmung der Säfte zu bewirken, wobei die Einrichtung ferner eine Verdampfungskammer (41) besitzt, in der die erhitzten Säfte im Gegenstrom zu einem Frischluftfluss, der sich mit Dampf aufläd, fliessen, wobei die konzentierten Säfte am Fuss des Verdampfers (41) aufgenommen und durch eine Rückführungsleitung zum Pufferbehälter (40) geführt werden, wobei der Fuss des Pufferbehälters (40) Extraktionsmittel für die festen Ablagerungen bildet, die sich abgesetzt haben und durch Fördermittel mit dem Mischer (12) verbunden sind, um die Einverleibung der abgelagerten Stoffe in das organisch-mineralische Düngemittel zu bewirken, und wobei der genannte Pufferbehälter (40) geeignet ist, eine Vergärungsreaktion in flüssiger Phase der genannten Säfte zu bewirken und an seinem Scheitel mit einem Kreis zu Abführung des Methans, das in seinem Inneren erzeugt worden ist, zum Speichergasometer (7) verbunden ist.

9. Einrichtung nach einem der Ansprüche 6 bis 8, dadurch gekennzeichnet, dass der genannte Mischer (12), der geeignet, ist die Homogeneiät des organisch-mineralischen Düngemittels (33) zu gewährleisten, ferner wenigstens einen Trichter (30, 31) besitzt, der eine Zusatzreserve wie ein mineralisches Salz enthält, das geeignet ist, die Zusammensetzung der Mischung zum Erhalt eines konstanten Gehalts an Wirkstoffen im Düngemittel einzustellen.

## Claims

1. Method for recovering and working solid residues or rejects of industrial, agricultural or domestic origin, of the type wherein the residues are subjected to a bacterial digestion step for the production of a recovered and stored fuel gas whereas the solid phase of the digestate from the previous step is subjected to an incineration in a furnace with a view to producing thermal energy and wherein one improves the incineration conditions by injecting into the furnace one part of the gas from the methanization phase and one improves the conditions of the methane production by using the heat of the furnace to reheat the magma being in the process of digestion and the method is further characterized by the sequence of the following steps:

a) one separates by sifting within the starting material the biggest particles which are intended to be carried towards the furnace from the finest particles which are intended to be conveyed towards the digestor with a view to perform the methanization reaction;

b) one mixes the phase of the said finest particles with a liquid phase consisting of the juices separated from the digestate issuing from the methanization digestor;

c) one lets the finest particles stay within the methanization digestor which receives a supply of calories to accelerate the reaction, the fuel gas produced being stored in a gasometer;

d) one separates through filtration within the digestate issuing from the reactor a solid phase from a liquid phase constituting the juices which are recycled according to the step b) hereinabove;

e) one subjects the solid phase of the digestate to a decompacting and sifting, the finest particles forming the passing phase are separated whereas the biggest particles or rejects are directed together with the rejects from the sifting according to the step a) towards the incineration furnace to constitute the fuel feeding the said furnace;

f) one controls at any time the parameters of the combustion within the said furnace, such as the temperature and one introduces into the furnace a metered quantity of fuel gas from the gasometer, this in accordance with verified data to complete the possible deficiencies in the calorific value of the main solid fuel thereby adjusting the conditions of the combustion;

g) one causes the combustion smokes from the said furnace to pass into a first heat recuperator such as a tubular boiler for instance or any other exchangers to produce a fluid at a high temperature for industrial purposes;

h) one washes the smokes when issuing from the first heat recuperator by neutralization by means of a basic milk originating from the combustion ashes suspended within an aqueous phase;

i) one collects the said milk at the outlet of the neutralization step according to h) hereinabove and one separates the solid phase which is mixed with the passing phase originating from the sifting according to step e) hereinabove to constitute an organo-mineral ameliorator for the soils;

j) the purified gases issuing from the neutralization step according to h) hereinabove are carried to a second heat recuperator delivering a heat-conveying fluid carried towards a heat exchanger located within the methanization reactor by thus constituting the supply of calories of the step c) hereinabove.

2. Method according to claim 1, characterized in that the thermal energy originating from the incineration phase and carried by the heat-conveying fluid originating from the second heat recuperator is used to obtain the concentration of the juices originating from the separation of the solid phase from the digestate with a view to recyle the said juices after concentration, which are incorporated into the starting material introduced into the digestor.

3. Method according to claim 1 hereinabove, further characterized in that the thermal energy originating from the incineration phase and contained within the heat-carrying fluid originating from the second heat recuperator is used to reheat and concentrate the liquid juices separated from the digestate which are introduced as a liquid phase into a second digestor or a secondary digestor for the production of an auxiliary flux of methane.

4. Method according to claim 1 hereinabove, characterized in that the finest particles forming the passing phase of the said sifting step e) hereinabove are directed towards a mixer wherein they are incorporated to the solid residues originating from a phase of sedimentation of the neutralization milk formed of the ashes suspended in an aqueous phase, these products within the mixer moreover receiving the solid residues originating from the settling of the sludges extracted from the digestate to constitute through the incorporation of these three elements a horticultural organo-mineral ameliorator or fertilizer.

5. Method according to claim 4 hereinabove, characterized in that the treated residues and rejects are further subjected to a step of removal of the solids consisting of glass objects or elements, this glass phase being subjected to a crushing until the obtainment of a powdery granulometry leading to the formation of a sandy phase which is introduced into the said mixture (12) to obtain a complete cultivation substrate.

6. Plant for carrying out the method according to one of claims 1 to 5 hereinabove, of the type comprising:

an anaerobic fermentation digestor (5) fed with solid waste or residues of industrial, agricultural or domestic origin with a view to perform the production of methane;

a gasometer (7) forming a storage vessel for the methane thus produced;

means (8) for the settling of the digestate from the digestor to separate the solid phase from a liquid phase;

sifting means (10) arranged downstream of the digestor and of the said settling means in order to provide the separation within the solid phase of the digestate, of the finest particles and of the coarsest elements or rejects;

a conveyor capable of carrying the said rejects constituting the fuel intended for the incineration;

an incineration furnace (16) provided with a post-combustion chamber (17) and comprising at least a gas burner (53) fed from the said gasometer;

a first heat recuperator (19) supplied with fuel gas from the incineration furnace and delivering a heat-conveying fluid at high temperature, and the plant is characterized in that it further comprises:

a second heat recuperator such as a condensor (25) located downstream of the first recuperator (19) on the circuit of the smokes and associated with a circuit of heat-carrying fluid at mean temperature;

a heat exchanger (38') incorporated into the digestor (5) and connected to the second heat recuperator and thus capable of transferring calories originating from the said second heat recuperator towards the magma being in the process of bacterial fermentation within the digestor;

a tank (21) for the preparation of a neutralization milk consisting of the combustion ashes originating from the incineration furnace and suspended in a liquid phase;

a neutralization chamber through which are flowing on the one hand the said neutralization milk and on the other hand the smokes issuing from the first heat recuperator (19) and carried towards the second heat recuperator (25), the meeting of the acid smokes with the milk containing the suspended ashes being capable of providing the neutralization of the said smokes;

a mixer (12) adapted to receive the particles passing at the sifting (10) located downstream of the digestor on the one hand and the solid extracts settled in the neutralization tank (21), this mixer being capable of delivering an organo-mineral composition usable as an agricultural or horticultural ameliorator or fertilizer (33).

7. Plant according to claim 6 hereinabove, characterized in that it comprises a unit for the concentration or settling of the juices originating from the filtration phase (9) at the outlet of the digestor and this unit comprises a heat exchanger (39) fed with the heat-conveying fluid originating from the second heat recuperator (25), this exchanger (39) being connected by an incoming pipeline (9) to the said filtration means (8) located downstream of the digestor (5) and by an outlet pipeline (4) leading to the supply (3) of the digestor with a view to recycle the concentrated juices for feeding and seeding the digestor.

8. Plant according to claim 7 hereinabove, characterized in that the said unit for the concentration of the juices further comprises a buffer tank (40) adapted to provide for the settling of the said juices, this tank being fed through the pipeline (9) from the filtration (8) at the outlet of the digestor and the buffer tank (40) feeds the said heat exchanger (39) to cause the reheating of the juices, the plant further comprising an evaporation vessel (41) in which the reheated juices are flowing in counter-current relationship with a flux of fresh air which loads itself with vapor, the concentrated juices being collected at the base of the evaporator (41) and carried through a recycling pipeline to the said buffer tank (40), the base of the buffer tank (40) comprising means for the extraction of the settled solid deposits and connected by conveying means to the said mixer (12) with a view to incorporate the said settled deposit into the organo-mineral fertilizer and the said buffer tank (40) adapted to provide for a digestion reaction in liquid phase of the said juices being connected with its top to a circuit for discharging the methane produced therein towards the storage gasometer (7).

9. Plant according to one of the claims 6 to 8, characterized in that the said mixer (12) adapted to provide for the homogeneousness of the organo-mineral fertilizer (33) further comprises at least one hopper (30, 31) containing a supply of additives such as a mineral salt suitable for adjusting the composition of the mixture with a view to provide for the constant richness of the fertilizer in active principles.

EP 0 250 475 B1